# EUROPÄISCHE PATENTANMELDUNG

(11) **EP 4 276 086 A1**
(43) Veröffentlichungstag der Anmeldung: **15.11.2023**
(21) Anmeldenummer: 22020213.9
(22) Anmeldetag: 10.05.2022
(51) Int. Cl.: C07C 41/01, C07C 43/04, C07C 29/15, C07C 31/04, B01D 3/14, B01D 5/00, B01D 53/14, C07C 41/09

(54) **VERFAHREN UND ANLAGE ZUR HERSTELLUNG VON DIMETHYLETHER**

(71) Anmelder: Linde GmbH, 82049 Pullach (DE)
(72) Erfinder: Winkler, Florian, 82049 Pullach (DE); Peschel, Andreas, 82049 Pullach (DE)
(74) Vertreter: Fischer, Werner

(57) **Zusammenfassung**

Ein Verfahren (100) zur Herstellung von Dimethylether wird vorgeschlagen, bei dem ein die Reaktanden Kohlenmonoxid und/oder Kohlendioxid und Wasserstoff enthaltendes Synthesegas unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch eine Syntheseeinheit (14, 4) geführt wird, in dem der Methanolkatalysator und der Dimethyletherkatalysator in einer gemeinsamen Katalysezone bereitgestellt sind, wobei der Syntheseeinheit (14, 4) ein Methanol, Dimethylether, Wasser und einen Teil der Reaktanden enthaltendes Rohprodukt entnommen wird, und wobei unter Verwendung zumindest eines Teils des Rohprodukts in einer Trenneinheit (150) eine überwiegend oder ausschließlich Wasser und Methanol enthaltende Fraktion bereitgestellt wird. Es ist vorgesehen, dass zumindest ein Teil der überwiegend oder ausschließlich Wasser und Methanol enthaltenden Fraktion in einem Wasser und Methanol enthaltenden Rückspeisestrom in die Syntheseeinheit (14, 4) zurückgeführt wird. Eine entsprechende Anlage ist ebenfalls Gegenstand der vorliegenden Erfindung.

## Beschreibung

Die Erfindung betrifft ein Verfahren und eine Anlage zur Herstellung von Dimethylether.

### Hintergrund

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen. Neuerdings wird Dimethylether zunehmend als Ersatz für Brenngas (Flüssiggas) und Kraftstoffe wie Diesel eingesetzt.

Dimethylether kann entweder direkt aus Methanol oder indirekt aus Erd- oder Biogas erzeugt werden. Im letzteren Fall wird das Erd- oder Biogas zunächst zu Synthesegas umgesetzt. Das Synthesegas wird anschließend entweder in einer zweistufigen Reaktion zu Methanol und anschließend zu Dimethylether oder in einer einstufigen Reaktion direkt zu Dimethylether konvertiert.

Unter Synthesegas soll hier ein Gasgemisch verstanden werden, das zumindest Kohlenmonoxid und Wasserstoff in wechselnden Anteilen, die zusammen jedoch den überwiegenden Teil des Gasgemischs ausmachen, enthält. Auch Kohlendioxid kann enthalten sein, welches durch eine reverse Wassergasshiftreaktion (RWGS) zu weiterem Kohlenmonoxid umgesetzt werden kann.

Synthesegas kann durch Pyrolyse von Kohle, Öl, kohlenstoffhaltigen Abfällen, Biomasse oder anderen Ausgangsstoffen, durch Trockenreformierung (Dry Reforming) von Erdgas mit Kohlendioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden. Auch eine Herstellung aus Kohlendioxid und Wasserstoff durch die bereits erwähnte reverse Wassergasshiftreaktion ist möglich.

Die Synthese von Dimethylether aus Synthesegas ist thermodynamisch und wirtschaftlich vorteilhaft gegenüber einer Synthese aus Methanol.

Die vorliegende Erfindung betrifft insbesondere die einstufige oder direkte Synthese von Dimethylether, worunter eine Synthese verstanden wird, bei der sämtliche Reaktionen in ein und demselben Reaktor und in ein und demselben Katalysatorbett ablaufen. Bei den ablaufenden Reaktionen handelt es sich um eine Reaktion von Wasserstoff mit Kohlenmonoxid und/oder Kohlendioxid zu Methanol und eine (Weiter-) Reaktion von Methanol zu Dimethylether und Wasser. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 100 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält typischerweise neben Dimethylether nicht umgesetzte Komponenten des Synthesegases sowie weitere Reaktionsprodukte. Typischerweise weist der Produktstrom neben Dimethylether zumindest Methanol, Wasser, Kohlendioxid, Kohlenmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlendioxid und leichter als Kohlendioxid siedende Komponenten wie Wasserstoff und Kohlenmonoxid enthalten. Je nach Produktspezifikation müssen diese zumindest teilweise abgetrennt werden.

Die Zusammensetzung des Produktstroms (dieser oder ein hieraus gebildetes Gasgemisch wird nachfolgend auch als Dimethylether enthaltendes Produktgemisch bezeichnet) wird dabei insbesondere durch die gewählten Reaktionsbedingungen während der Synthese bestimmt. Je näher diese Zusammensetzung stromab des Reaktors an der geforderten Spezifikation liegt, desto geringer ist der Aufwand, der zur Aufreinigung des Produktstroms betrieben werden muss.

Insbesondere in Bezug auf die hier verwendete Terminologie, aber auch auf grundsätzliche Überlegungen die (insbesondere direkte) Synthese von Dimethylether aus Synthesegas betreffend, sei auf den Artikel von Kiendl et al., Chem. Ing. Tech. 2020, 92, No. 6, 736-745, verwiesen.

In der Praxis weisen sowohl bekannte zweistufige Reaktionsverfahren zur Herstellung von Dimethylether als auch einstufige Verfahren gewisse Nachteile auf, die die vorliegende Erfindung überwinden will.

### Offenbarung der Erfindung

Diese Aufgabe wird durch ein Verfahren und eine Anlage mit den Merkmalen der jeweiligen unabhängigen Patentansprüche gelöst. Vorteilhafte Ausgestaltungen der sind Gegenstand der Unteransprüche sowie der nachfolgenden Beschreibung.

Je nach Stöchiometriezahl des bei der einstufigen Dimethylethersynthese frisch eingesetzten Synthesegases entstehen entweder (vorwiegend) Dimethylether und Wasser (bei einer Stöchiometriezahl von 2 und mehr) oder es entstehen (vorwiegend) Dimethylether und Kohlendioxid (bei einer Stöchiometriezahl von unter 2 und in Richtung 1). Im letzteren Fall wird Kohlendioxid in der Regel mittels einer chemischen Wäsche abgetrennt und zur Synthesegaserzeugung rezykliert oder verworfen.

Zum Begriff der Stöchiometriezahl sei auf Fachliteratur und insbesondere den eingangs zitierten Artikel von Kiendl et al. verwiesen.

Die bei einer Stöchiometriezahl von 2 und mehr bei der Dimethylethersynthese ablaufenden (Netto-)Reaktionen sind dabei die folgenden: 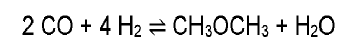 Δ H₂₉₈ = -205 kJ/mol 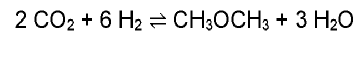 Δ H₂₉₈ = -123 kJ/mol

Bei einer Stöchiometriezahl von 1 läuft die folgende (Netto-)Reaktion ab: 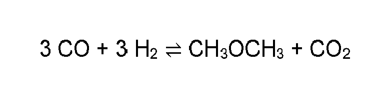 Δ H₂₉₈ = -246 kJ/mol

Im Falle eines Synthesegaseinsatzes mit einer Stöchiometriezahl von weniger als 2 muss typischerweise Kohlendioxid aus dem System entfernt werden. Eine Möglichkeit dazu besteht durch einen Abzug von Kohlendioxid aus einer zur Bearbeitung des Produktgemischs der Dimethylethersynthese eingesetzten Trennkolonne, wie beispielsweise in der EP 3 521 267 A1 oder der EP 3 960 265 A1 beschrieben.

Allerdings entsteht aufgrund der Gleichgewichtslage auch bei sehr geringen Verhältnissen von Wasserstoff zu Kohlenmonoxid immer ein Teil Wasser. Dieses Wasser muss herkömmlicherweise aufwendig von einem Methanolrecycle zur Dimethylethersynthese abgetrennt werden. Die bisher eingesetzten Katalysatoren sind oft anfällig gegenüber einem hohen Wasserpartialdruck, da sie typischerweise in Gegenwart von Wasser (und/oder Kohlendioxid) sintern. Sie können also nur begrenzte Mengen an Wasser tolerieren bzw. bedeuten hohe Wasserpartialdrücke i.d.R. eine beschleunigte Alterung des Katalysators.

Zusätzlich erfordert diese Wasserbildung auch immer ein Verhältnis von Wasserstoff zu Kohlenmonoxid im frisch eingesetzten Synthesegas von mehr als 1. Dieses ist insbesondere im Falle einer Vergasung von z.B. Biomasse, Petcoke, Schweröl oder Kohle aufwendig zu erzeugen, da diese Synthesegase oft ein Verhältnis von Wasserstoff zu Kohlenmonoxid von weniger als 1 aufweisen. Die hierdurch unvermeidliche Wasserbildung ist insbesondere aus den genannten Gründen unvorteilhaft.

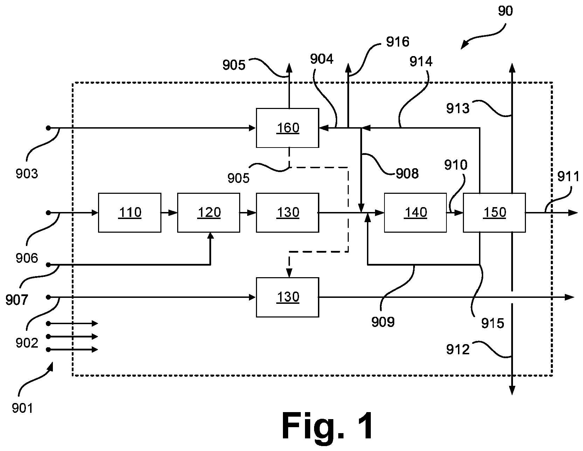

Mit anderen Worten werden bei der Vergasung von z.B. Kohle-, Petcoke, Biomasse oder Schweröl, aber auch bei der partiellen Oxidation oder der autothermen Reformierung von Einsatzstoffen schwerer als Erdgas oder bei der Verwendung von Synthesegasen aus Stahlwerken (z.B. Hochofen-, Konverter- und Koksofengas) oft Synthesegase mit niedrigem Verhältnis von Wasserstoff zu Kohlenmonoxid erzeugt. Das Verhältnis liegt oft unterhalb von 1. Damit solche Synthesegase für die Synthese von Dimethylether eingesetzt werden können, wird häufig ein Teil des Gases geshiftet um zusätzlichen Wasserstoff zu erzeugen und das auf diese Weise gebildete Kohlendioxid wird entfernt und verworfen. Ein entsprechendes Verfahren ist in Figur 1 veranschaulicht, die unten näher erläutert wird.

Bei einem entsprechenden Verfahren entstehen einerseits hohe Kohlendioxidemissionen und andererseits ein hoher apparativer und energetischer Aufwand für die Shifteinheit und die erforderliche Kohlendioxidwäsche.

In herkömmlichen Verfahren können mittels einer Wassergasshiftreaktion bearbeitete und unbearbeitete Synthesegasanteile (zuvor und nachfolgend auch als "geshiftetes" und "ungeshiftetes" Synthesegas bezeichnet) vereinigt und anschließend zusammen mit einem Synthesegasrecycle zur einstufigen Synthese von Dimethylether geleitet werden. Dort setzen sich Wasserstoff und Kohlenmonoxid gemäß folgender Reaktionen (Einzelreaktionen der oben angegebenen Reaktionsgleichungen; MeOH steht für Methanol und DME für Dimethylether) um:

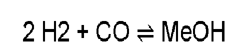

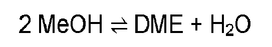

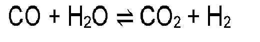

Es handelt sich hierbei um Gleichgewichtsreaktionen, die je nach Druck, Temperatur und Feedzusammensetzung eine andere Produktzusammensetzung ergeben. Die jeweiligen Nettoreaktionen für unterschiedliche Stöchiometriezahlen wurden bereits oben erläutert und sind dort im Einzelnen angegeben.

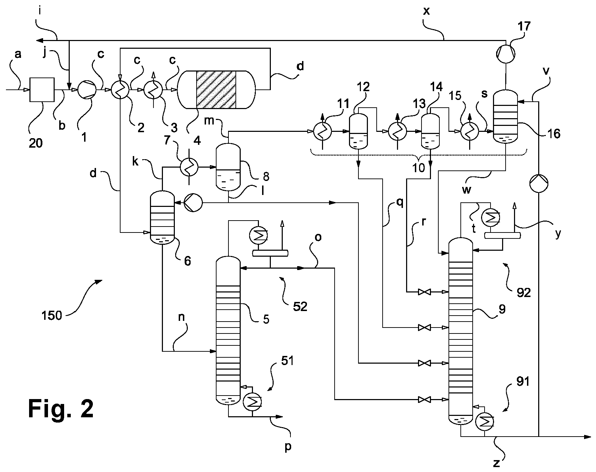

Eine Trennsequenz, die der vorliegenden Erfindung zugrunde liegen kann, ist in Figur 2 gezeigt und unten erläutert. Nach der Synthese von Dimethylether wird das Rohprodukt hier abgekühlt, z.B. durch Wärmeintegration mit dem Reaktionseinsatzgas, und in eine Absorptionskolonne geleitet. Das Sumpfprodukt dieser Absorptionskolonne wird in einer (ersten) Destillationssäule von Dimethylether und weiteren gelösten Gasen befreit, so dass ein überwiegend oder ausschließlich Methanol und Wasser enthaltender Sumpfstrom bzw. eine entsprechende Fraktion gebildet wird. Generell soll hier der Begriff "überwiegend" für einen Mol- oder Volumenanteil von wenigstens 50, 60, 70, 80, 90, 95 oder 99 Prozent stehen.

In herkömmlichen Verfahren wird nun die überwiegend oder ausschließlich Methanol und Wasser enthaltende Fraktion aufgetrennt und (nur) das Methanol zur Dimethylethersynthese rezykliert. Wasser wird aus dem System entfernt.

Ein Kopfstrom der ersten Destillationssäule kann stufenweise abgekühlt werden, wodurch dimethyletherreiche Kondensate gebildet werden. Diese können in eine weitere (zweite) Destillationssäule eingespeist werden. In der zweiten Destillationssäule wird Dimethylether als Sumpfprodukt, z.B. mit Kraftstoffspezifikation, erhalten. Am Kopf der zweiten Destillationssäule fallen Kohlendioxid und leichtere gelöste Gase wie Wasserstoff und Kohlenmonoxid an. Dieser Strom kann teilweise kondensiert werden. Das nichtkondensierte Gas kann zum Dimethyletherreaktor rezykliert oder ausgeleitet werden. Um Kohlendioxid aus einem Gasrecycle zum Dimethyletherreaktor zu entfernen, kann eine Wäsche mit Dimethylether erfolgen. Dies ist vorteilhaft, weil dadurch der Kohlendioxidgehalt im Dimethyletherreaktor niedrig und der Umsatz hochgehalten werden kann.

Die vorliegende Erfindung beruht nun auf der Erkenntnis, dass vorteilhafterweise zumindest ein Teil der erwähnten, überwiegend oder ausschließlich Wasser und Methanol enthaltenden Fraktion in den Dimethyletherreaktor zurückgeführt werden kann. Sie schlägt vor diesem Hintergrund ein Verfahren zur Herstellung von Dimethylether vor, bei dem ein die Reaktanden Kohlenmonoxid und/oder Kohlendioxid und Wasserstoff enthaltendes Synthesegas unter teilweiser Umsetzung der Reaktanden und Bildung von Methanol an einem oder mehreren Methanolkatalysatoren sowie Umsetzung zumindest eines Teils des Methanols an einem oder mehreren Dimethyletherkatalysatoren durch eine Syntheseeinheit geführt wird, in dem der Methanolkatalysator und der Dimethyletherkatalysator in einer gemeinsamen Katalysezone bereitgestellt sind. Die vorliegende Erfindung betrifft also eine direkte bzw. einstufige Synthese von Dimethylether.

Vorliegend wird unter einer Methanolreaktion bzw. einem Methanolkatalysator die Reaktion zu Methanol bzw. der dabei verwendete Katalysator bzw. unter einer Dimethyletherreaktion bzw. einem Dimethyletherkatalysator die Reaktion zu Dimethylether bzw. der dabei verwendete Katalysator verstanden.

Der Syntheseeinheit wird ein Methanol, Dimethylether, Wasser und einen Teil der Reaktanden enthaltendes Rohprodukt entnommen, wobei unter Verwendung zumindest eines Teils des Rohprodukts in einer Trenneinheit eine überwiegend oder ausschließlich Wasser und Methanol enthaltende Fraktion bereitgestellt wird. Hierbei ist vorgesehen, dass zumindest ein Teil der überwiegend oder ausschließlich Wasser und Methanol enthaltenden Fraktion in einem Wasser und Methanol enthaltenden Rückspeisestrom in die Syntheseeinheit zurückgeführt wird.

Durch den Einsatz der vorliegenden Erfindung kann insbesondere der Aufwand für eine Wassergasshift und Kohlendioxidwäsche (insbesondere eine sogenannte Rectisoleinheit) deutlich reduziert oder ganz vermieden werden. Dies ergibt sich direkt aus der Rückführung des entstehenden Wassers aus der Synthese, oder eines Teils hiervon, in den Dimethyletherreaktor. Auf eine Trennung von Methanol, das ebenfalls zurückgeführt wird, kann verzichtet werden.

Durch den Einsatz der vorliegenden Erfindung kann erreicht werden, dass das Wasser aus dem Recycle mit Kohlenmonooxid aus einem Frischeinsatz im Dimethyletherreaktor ähnlich wie in einer Shifteinheit zu Wasserstoff und Kohlendioxid umgesetzt werden kann. Auf diese Weise erhöht sich der Wasserstoffgehalt und damit das Verhältnis von Wasserstoff zu Kohlenmonoxid, so dass im Rahmen der vorliegenden Erfindung auch die zuvor genannten Einsätze einfach verwendet werden können. Für eine möglichst effiziente Wasserstoffgenerierung wird vorteilhafterweise der Gehalt im Frischeinsatz und Recycle entsprechend niedrig gehalten.

Im Rahmen der vorliegenden Erfindung kann der Syntheseeinheit neben dem Synthesegas und dem Rückspeisestrom Frischwasser zugeführt werden. Auf diese Weise können der Wassergehalt und damit dessen positive Effekte, insbesondere die zusätzliche Wasserstoffbildung, weiter erhöht werden.

In einer Ausgestaltung der Erfindung wird unter Verwendung zumindest eines Teils des Rohproduktstroms in der Trenneinheit eine überwiegend oder ausschließlich die Edukte enthaltende Fraktion bereitgestellt, die ebenfalls zumindest zum Teil in die Syntheseeinheit zurückgeführt wird. Es handelt sich also um einen Recycle von nicht umgesetztem Synthesegas. Kohlendioxid kann zumindest teilweise aus einer derartigen, überwiegend oder ausschließlich die Edukte enthaltenden Fraktion entfernt werden, beispielsweise durch eine geeignete Wäsche.

In einer Ausgestaltung der Erfindung weist die Syntheseeinheit einen ersten Reaktor oder Teilreaktor und einen zweiten Reaktor oder Teilreaktor auf, wobei zwischen dem ersten Reaktor oder Teilreaktor und dem zweiten Reaktor eine Abscheideeinheit verwendet wird, unter Verwendung derer ein Partialdruck von Wasser und Kohlendioxid reduziert wird. Diese Abscheideeinheit kann insbesondere für eine Zwischenkondensation (insbesondere durch Abkühlung) und/oder eine Auswaschung von Kohlendioxid mittels Dimethylether oder einem Methanol-Wasser-Gemisch eingerichtet sein. Durch den Einsatz einer derartigen Abscheideeinheit können insbesondere geringere Partialdrücke von Wasser und Kohlendioxid im darauffolgenden Reaktor eingestellt werden. Dies erhöht die Lebensdauer des Katalysators. Die Entfernung von Kohlendioxid gibt zusätzlich Shiftkapazitäten frei, die für eine zusätzliche Flexibilität in der Einsatzstoffzusammensetzung genutzt werden können.

In einer Ausgestaltung der Erfindung wird das Synthesegas zu einem ersten Anteil unter Verwendung einer Vergasungseinheit, bereitgestellt, und das Synthesegas umfasst ferner separat von der Vergasungseinheit bereitgestellten und/oder unter Verwendung einer Wassergasshiftreaktion bereitgestellten Wasserstoff. Die Vergasungseinheit kann insbesondere zur Vergasung von Einsatzstoffen wie Erdgas, aber auch der zuvor erwähnten Einsatzstoffe Biomasse, Petcoke, Schweröl, kohlenstoffhaltigem Abfall oder Kohle eingerichtet sein.

In einer Ausgestaltung der Erfindung wird der separat von der Kohlenwasserstoffvergasungseinheit bereitgestellte Wasserstoff zumindest zu einem Teil mittels Elektrolyse bereitgestellt. Hierbei kann insbesondere auf regenerative Energiequellen zurückgegriffen werden, so dass sich der Kohlendioxidfußabdruck des vorgeschlagenen Verfahrens in einer entsprechenden Ausgestaltung verringert.

In einer Ausgestaltung der Erfindung wird die überwiegend oder ausschließlich Wasser und Methanol enthaltende Fraktion derart gebildet, dass sie einen Summengehalt von Wasser und Methanol von mehr als 90% aufweist.

In einer Ausgestaltung des erfindungsgemäßen Verfahrens wird die überwiegend oder ausschließlich Wasser und Methanol enthaltende Fraktion unter Verwendung einer Absorptionskolonne und einer Destillationssäule bereitgestellt, wobei der Absorptionskolonne zumindest ein Teil des Rohprodukts zugeführt wird, ein überwiegend leichter als Methanol und Wasser siedende Komponenten enthaltender Kopfstrom der Absorptionskolonne zu einem Teil kondensiert und als Rücklauf auf die Absorptionskolonne zurückgeführt wird, ein überwiegend Methanol und Wasser sowie Kohlendioxid enthaltender Sumpfstrom der Absorptionskolonne in die Destillationssäule eingespeist wird, und die überwiegend oder ausschließlich Wasser und Methanol enthaltende Fraktion unter Verwendung eines Sumpfstroms aus der Destillationssäule bereitgestellt wird.

Der Rückspeisestrom kann in einer Ausgestaltung der vorliegenden Erfindung zumindest einen Teil der überwiegend oder ausschließlich Wasser und Methanol enthaltenden Fraktion enthalten, der einer Verdampfung und Druckbeaufschlagung unterworfen wird. Hierbei sind die nachfolgenden Varianten möglich.

In einer Ausgestaltung kann die Druckbeaufschlagung vor der Verdampfung mittels einer Pumpe durchgeführt werden. Mit anderen Worten wird die überwiegend oder ausschließlich Wasser und Methanol enthaltende Fraktion oder ein entsprechender Teil hiervon also flüssig druckbeaufschlagt.

Die Verdampfung kann hierbei insbesondere zumindest zum Teil ein Durchleiten eines heißen Gasstroms durch die überwiegend oder ausschließlich Wasser und Methanol enthaltende Fraktion, insbesondere eines rückgeführten Synthesegasstroms, umfassen. Auf diese Weise ist eine besonders energieeffiziente Verdampfung möglich.

Alternativ dazu kann in weiteren Ausgestaltungen die Druckbeaufschlagung nach der Verdampfung mittels eines Verdichters durchgeführt werden. Mit anderen Worten wird die überwiegend oder ausschließlich Wasser und Methanol enthaltende Fraktion oder ein entsprechender Teil hiervon also gasförmig druckbeaufschlagt.

Das Synthesegas kann in Ausgestaltungen der Erfindung mit einem Verhältnis von Wasserstoff zu Kohlenmonoxid von weniger als 2 oder weniger als 1 bereitgestellt werden. Mit anderen Worten ermöglicht die vorliegende Erfindung durch die vorgeschlagene Rückführung von Wasser und die hierdurch bewirkte Wasserstoffbildung auch die vorteilhafte Nutzung der eingangs erwähnten alternativen Vergasungseinsatzstoffe.

Die Erfindung erstreckt sich auch auf eine Anlage zur Herstellung von Dimethylether, mit einer Syntheseeinheit und Mitteln, die dafür eingerichtet sind, ein die Reaktanden Kohlenmonoxid und/oder Kohlendioxid und Wasserstoff enthaltendes Synthesegas unter teilweiser Umsetzung der Reaktanden und Bildung von Methanol an einem oder mehreren Methanolkatalysatoren sowie Umsetzung zumindest eines Teils des Methanols an einem oder mehreren Dimethyletherkatalysatoren durch die Syntheseeinheit zu führen, wobei in der Syntheseeinheit der Methanolkatalysator und der Dimethyletherkatalysator in einer gemeinsamen Katalysezone bereitgestellt sind, und wobei die Anlage Mittel aufweist, die dafür eingerichtet sind, aus der Syntheseeinheit ein Methanol, Dimethylether, Wasser und einen Teil der Reaktanden enthaltendes Rohprodukt zu entnehmen und unter Verwendung zumindest eines Teils des Rohprodukts in einer Trenneinheit eine überwiegend oder ausschließlich Wasser und Methanol enthaltende Fraktion bereitzustellen.

Hierbei ist vorgesehen, dass die Anlage dafür eingerichtet ist, zumindest einen Teil der überwiegend oder ausschließlich Wasser und Methanol enthaltenden Fraktion in einem Wasser und Methanol enthaltenden Rückspeisestrom in die Syntheseeinheit zurückzuführen.

Zu Merkmalen und Vorteilen einer entsprechenden Anlage und möglicher Ausgestaltungen sei auf die obigen Erläuterungen bezüglich des erfindungsgemäßen Verfahrens und seiner Ausgestaltungen ausdrücklich verwiesen.

Anlagen gemäß Ausgestaltungen der vorliegenden Erfindung weisen insbesondere Mittel auf, die sie zur Durchführung eines vorstehend beschriebenen Verfahrens und entsprechender Ausgestaltungen ertüchtigen. Die erfindungsgemäße Anlage bzw. deren vorteilhafte Ausgestaltungen profitieren dementsprechend von den Vorteilen des jeweils entsprechenden Verfahrens in analoger Weise und umgekehrt.

Im Folgenden werden weitere Merkmale und Vorteile der vorliegenden Erfindung anhand von Ausführungsbeispielen unter Bezugnahme auf die beiliegende Zeichnung näher erläutert.

### Kurze Beschreibung der Figuren

Figur 1 zeigt eine nicht erfindungsgemäße Ausgestaltung eines Verfahrens zur Herstellung von Dimethylether in stark vereinfachter Darstellung.
Figur 2 zeigt ein Verfahren zur Herstellung von Dimethylether, das einer Ausgestaltung der Erfindung zugrunde liegen kann, in Form eines Prozessflussdiagramms.
Figur 2 zeigt ein Verfahren zur Herstellung von Dimethylether gemäß einer Ausgestaltung der Erfindung in Form eines Prozessflussdiagramms.

### Ausführungsform(en) der Erfindung

In den Figuren werden Verfahrensschritte und Vorrichtungskomponenten teilweise gemeinsam beschrieben bzw. mit identischen Bezugszeichen angegeben. Generell betreffen Erläuterungen zu Verfahrensschritten die entsprechenden Vorrichtungskomponenten in gleicher Weise und umgekehrt. Baulich oder funktionell gleiche oder vergleichbare Komponenten sind ggf. mit identischen Bezugszeichen angegeben.

In Figur 1 ist eine nicht erfindungsgemäße Ausgestaltung eines Verfahrens zur Herstellung von Dimethylether stark vereinfacht veranschaulicht und insgesamt mit 90 bezeichnet. Komponenten des Verfahrens 90 bzw. Apparate oder Anlagenteile, die hierin verwendet werden, sind ein Schutzbett 110, eine Mischeinheit 120, eine Verdichtungseinheit 130, eine Syntheseeinheit 140 zur Direktsynthese von Dimethylether und eine Trenneinheit 150. Ferner sind eine befeuerte Heizeinheit 160 und ein Dampfsystem 130 dargestellt.

Dem Verfahren insgesamt werden, wie insgesamt mit den Pfeilen 901 bezeichnet, beispielsweise Energie, Kühlwasser und Niederdruckdampf zugeführt. In das Dampfsystem wird Kesselspeisewasser 902 eingespeist, der Heizeinheit 160 werden im dargestellten Beispiel Verbrennungsluft 903 und rückgeführtes Synthesegas 904 sowie ggf. Brenngas (nicht veranschaulicht) zugeführt. Verbrennungsabgase 905 werden aus dem Verfahren 90 ausgeleitet, in der Heizeinheit 160 gebildete Wärme wird dem Dampfsystem 130 zugeführt.

In die Mischeinheit 120 werden über das Schutzbett 110 frisches, ungeshiftetes Synthesegas 906 sowie separat dazu geshiftetes Synthesegas 907 geführt. Auf diese Weise können in der Mischeinheit die gewünschten Eigenschaften des Synthesegases eingestellt werden, das über die Verdichtungseinheit 130 nach Vereinigung mit rückgeführtem Synthesegas 908 und rückgeführtem Methanol 909 in die Syntheseeinheit 140 eingespeist wird. Ein dort gebildeter Rohproduktstrom 910 wird in die Trenneinheit 150 geführt, wo ein Dimethyletherprodukt 911 von Wasser 912, einem Stoffstrom 913, nicht umgesetztem Synthesegas 914 und Methanol 915 getrennt wird. Bei dem Stoffstrom 913 handelt es sich insbesondere um einen an Kohlendioxid reichen Strom, der aus dem Verfahren ausgeführt wird (Purgestrom). Entsprechendes Kohlendioxid kann insbesondere durch eine unterstöchiometrische Zugabe im Einsatz (also bei einem Verhältnis von Wasserstoff zu Kohlendioxid von weniger als 2) entstehen. In solchen Fällen reagiert ein Teil des Einsatzes zu Kohlendioxid, welches aus dem System abgeführt werden sollte. Das nicht umgesetzte Synthesegas wird in Teilen wie bereits zu 904 und 908 erläutert zurückgeführt. Ein weiterer Teil 916 kann aus dem Verfahren 90 ausgeleitet werden. Das abgetrennte Methanol wird, wie bereits zu 909 erläutert, rückgeführt.

In Figur 2 ist ein Verfahren zur Erzeugung von Dimethylether, das einer Ausgestaltung der Erfindung zugrunde liegen kann, in Form eines vereinfachten Prozessflussdiagramms schematisch dargestellt.

Zusätzlich zu den in Figur 1 veranschaulichten Komponenten wird hier eine Synthesegaserzeugungseinheit 20 verwendet, die mit einem geeigneten Einsatz a, beispielsweise Erd- oder Biogas, beschickt werden kann, und der ein Synthesegasstrom b entnommen werden kann.

Der Synthesegasstrom b kann, gegebenenfalls nach einer Beimischung weiterer Ströme und Einstellen seiner Zusammensetzung, beispielsweise mittels einer Mischeinheit 120 wie in Figur 1 veranschaulicht, mittels der hier mit 1 bezeichneten Verdichtereinheit, die der Verdichtereinheit 130 gemäß Figur 1 entsprechen kann, druckerhöht werden. Hierdurch kann ein für eine nachfolgende einschrittige Dimethylethersynthese erforderlicher Druck, beispielsweise ein Druck von 20 bis 80 bar, eingestellt werden.

Ein entsprechend verdichteter Strom, nun mit c bezeichnet, wird durch einen ersten Wärmetauscher 2 geführt, der mit einem Produktstrom d eines Reaktors 4 zur Synthese von Dimethylether (siehe unten, beispielsweise entsprechend der Syntheseeinheit 140 gemäß Figur 1) erwärmt werden kann. Der entsprechend erwärmte Strom, weiter mit c bezeichnet, weist stromab des ersten Wärmetauschers 2 beispielsweise eine Temperatur von 200 bis 300 °C auf. Der Strom c wird gegebenenfalls durch einen zweiten Wärmetauscher 3 geführt, welcher auch als Spitzenerhitzer bezeichnet wird.

Der in dem zweiten Wärmetauscher 3 weiter erwärmte Strom, auch hier weiter mit c bezeichnet, wird in eine hier mit 4 bezeichnete Syntheseeinheit zur Herstellung von Dimethylether eingespeist, die als Rohrreaktor ausgebildet ist, dessen Reaktionsrohre mit einem geeigneten Katalysator(gemisch) zur Einschrittsynthese von Dimethylether befüllt sind. Die Darstellung in der Figur 1 ist stark vereinfacht. Typischerweise sind Reaktoren bzw. Syntheseeinheiten 4 zur Synthese von Dimethylether stehend angeordnet, wobei ein Strom c bodenseitig in die Syntheseeinheit 4 eingespeist wird. Kopfseitig wird der Syntheseeinheit 4 ein Strom d entnommen.

Aufgrund der exothermen Reaktion in der Syntheseeinheit 4 liegt der Strom d bei nochmals höherer Temperatur vor. Der Strom d wird als Heizmedium durch den Wärmetauscher 2 geführt. Er kühlt sich hierdurch auf eine Temperatur ab, die beispielsweise ca. 30 °C oberhalb der Temperatur des Stroms c stromab des Verdichters 1 liegt. Der entsprechend abgekühlte Strom, weiter mit d bezeichnet, wird einer wie oben mit 150 bezeichneten Trenneinheit zugeführt. In der Trenneinheit 150 wird der Strom d zunächst in eine erste Absorptionskolonne, die zur Abtrennung von Methanol und/oder Wasser eingesetzt wird, und die in der Figur 2 mit 6 bezeichnet ist, eingespeist. In der Absorptionskolonne 6 aufsteigende Dämpfe werden durch einen am Kopf der Absorptionskolonne 6 aufgegebenen Rücklauf gewaschen, so dass sich am Kopf der Absorptionskolonne 6 die leichter flüchtigen und im Sumpf der Absorptionskolonne 6 die schwerer flüchtigen Komponenten anreichern.

Vom Kopf der Absorptionskolonne 6 wird ein Kopfstrom k entnommen und in einem Wärmetauscher 7 gegen ein geeignetes Kältemittel, beispielsweise Kühlwasser, gekühlt. Der entsprechend abgekühlte Strom k wird in einen Abscheiderbehälter 8 überführt, aus dessen Sumpf ein flüssiger Strom I entnommen und mittels einer nicht gesondert bezeichneten Pumpe zumindest zum Teil als Rücklauf auf die Absorptionskolonne 6 aufgegeben.

Enthält der Strom d im dargestellten Beispiel neben Dimethylether auch Methanol, Wasser, Kohlendioxid, Kohlenmonoxid und Wasserstoff (neben Spuren anderer Verbindungen wie oben erläutert), gehen hiervon aufgrund der erläuterten Rückwaschung Dimethylether, Kohlendioxid, Kohlenmonoxid und Wasserstoff in den Kopfstrom k über. Durch eine geeignete Abkühlung in dem Wärmetauscher 7 und entsprechende Abscheidebedingungen in dem Abscheiderbehälter 8 scheidet sich in dem Abscheiderbehälter 8 ein Sumpfprodukt ab, das im Wesentlichen aus Dimethylether und Kohlendioxid (gegebenenfalls mit Spuren von Methanol) besteht.

Vom Kopf des Abscheiderbehälters 8 kann ein Strom m gasförmig abgezogen werden, der zusätzlich zu Kohlendioxid, Kohlenmonoxid und Wasserstoff ebenfalls noch Dimethylether enthält. Der Strom m wird anschließend einer sequenziellen Abkühlung und Kondensation unterworfen, wie unten erläutert. Der nicht als flüssiger Rücklauf auf die Absorptionskolonne 6 aufgegebene Anteil des Stroms I wird, wie die bei der sequenziellen Abkühlung und Kondensation des Strom m anfallenden Kondensate, in eine Destillationssäule 9, die hier als Dimethylether-Kohlendioxid-Destillationssäule 9 bezeichnet wird, eingespeist.

Es sei ausdrücklich betont, dass die spezifische Bereitstellung des Stroms k, der aus dem Produktstrom d gewonnen wird, nicht in der dargestellten Weise erfolgen muss. Auch andere Möglichkeiten zur Abtrennung von Wasser und/oder Methanol sind einsetzbar, sofern sie zur Erzeugung eines Gasgemischs auf dem zuvor erwähnten ersten Druckniveau und dem ersten Temperaturniveau und mit den angegebenen Gehalten an den einzelnen Komponenten führen.

Es ist beispielsweise auch möglich, auf die Destillationssäule 5 zu verzichten und Wasser auf andere Art und Weise aus dem Strom n abzutrennen. Insbesondere wird das Wasser also mit in die Dimethylether-Kohlendioxid-Destillationssäule 9 eingespeist. In solchen Fällen wird das Wasser ggf. in einer der Dimethylether-Kohlendioxid-Destillationssäule 9 nachgelagerten Anlagenkomponente aus dem Dimethylether-Produkt z abgetrennt. In Ausgestaltungen der Erfindung wird jedoch eine überwiegend oder ausschließlich Wasser und Methanol enthaltende Fraktion gebildet.

Aus dem Sumpf der Absorptionskolonne 6 wird ein flüssiger Strom n entnommen und in geeigneter Höhe in die Destillationssäule 5 eingespeist, welche mit einem Sumpfverdampfer 51 und einem Kopfkondensator 52 betrieben wird. Der Strom n enthält im dargestellten Beispiel den überwiegenden Anteil des in dem Strom d enthaltenen Wassers und Methanols.

Der Sumpfverdampfer 51 und der Kopfkondensator 52 werden mit geeigneten, vorzugsweise in einer entsprechenden Anlage vorhandenen, Heiz- bzw. Kühlmedien betrieben. In dem Sumpfverdampfer 51 wird ein flüssiger, aus dem Sumpf der Destillationssäule 5 abgezogener Strom zum Teil verdampft und in einen unteren Bereich der Destillationssäule 5 eingespeist. Ein nicht verdampfter Anteil kann als Strom p abgezogen werden. Dieser kann insbesondere dem in Figur 1 veranschaulichten Wasser 912 entsprechen.

Vom Kopf der Destillationssäule 5 wird ein gasförmiger Strom abgezogen, zum Teil in dem Kopfkondensator 52 der Destillationssäule 5 verflüssigt und als flüssiger Rücklauf in einem oberen Bereich erneut in die Destillationssäule 5 eingespeist. Ein gasförmig verbleibender Anteil o wird abgezogen.

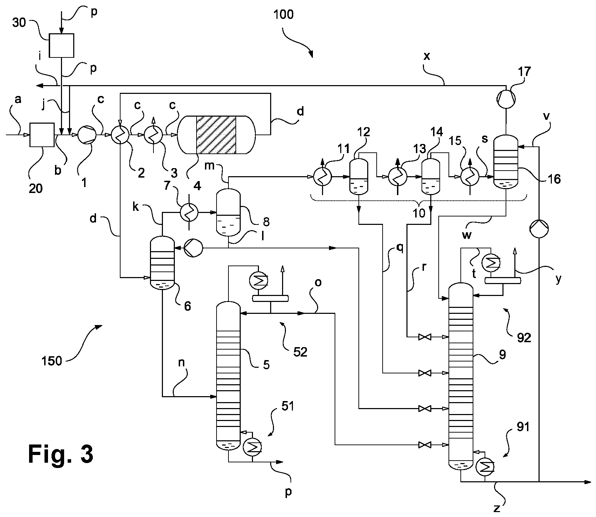

Aus dem Strom n, der im Wesentlichen noch Wasser, Methanol, Wasserstoff, Dimethylether und Kohlendioxid enthält, wird also in der Destillationssäule 5 ein im Wesentlichen Dimethylether und Kohlendioxid enthaltender Strom (Strom o) und ein im Wesentlichen Methanol und Wasser enthaltender Strom (Strom p) gebildet. Der Strom p kann in Ausgestaltungen der Erfindung in die Syntheseeinheit 140 bzw. 4 zurückgeführt werden, wie bereits erläutert und zu Figur 3 veranschaulicht.

Die Destillationssäule 5 kann auch so betrieben werden, dass im Wesentlichen kein Wasser, jedoch Methanol in nicht zu vernachlässigender Menge in das Kopfprodukt o übergeht. Dies ist beispielsweise vorteilhaft, wenn der Dimethylether für Einsatzzwecke verwendet werden soll, bei denen Methanol bei der Verwendung nicht stört. So kann eine höhere Ausbeute bezogen auf das eingesetzte Synthesegas erzielt werden.

Rücklaufmenge und Bodenzahl der Absorptionskolonne 6 können so optimiert werden, dass ein entsprechendes Sumpfprodukt n in möglichst geringer Menge anfällt. Vorteilhafterweise wird der Rücklauf, der als Anteil des Stroms I auf die Absorptionskolonne 6 aufgebracht wird, derart eingestellt, dass der Methanol- und/oder Wassergehalt in dem Strom k minimiert wird.

Schritte zur Weiterbehandlung des Stroms m sind hier insgesamt mit 10 angegeben. Der Strom m wird dabei zunächst einem Wärmetauscher 11 zugeführt und anschließend in einen Abscheiderbehälter 12 eingespeist. Die Abkühlung in dem Wärmetauscher 11 wird dabei derart vorgenommen, dass sich in dem Abscheiderbehälter 12 ein erstes Kondensat q abscheidet. Ein in dem Abscheiderbehälter 12 gasförmig verbleibender Anteil wird einem Wärmetauscher 13 zugeführt und anschließend in einen weiteren Abscheiderbehälter 14 eingespeist. Auch dort wird ein Kondensat, hier mit r bezeichnet, erhalten.

Die Kondensate q und r werden, zusammen mit dem nicht auf die Absorptionskolonne 6 rückgeführten Anteil des Stroms I, in die bereits erwähnte Dimethylether-Kohlendioxid-Destillationssäule 9 eingespeist, die wie unten erläutert betrieben wird. Auch der Strom o gelangt im hier veranschaulichten Beispiel dorthin. Ein auch am Kopf des Abscheiderbehälters 14 gasförmig verbleibender Anteil wird in einem weiteren Wärmetauscher 15 abgekühlt. Dieser Strom, hier mit s bezeichnet, liegt stromab des Wärmetauschers 15 auf einem Temperaturniveau knapp oberhalb des Schmelzpunkts von Kohlendioxid (bei dem vorherrschenden Druck) vor. Die Temperatur des Stroms m stromauf des Wärmetauschers 11 beträgt demgegenüber beispielsweise +35 °C. Der entsprechend abgekühlte Strom s wird in eine weitere Absorptionskolonne 16 überführt, die wie unten erläutert betrieben werden kann.

Auch eine stark vereinfachte Anordnung kann zum Einsatz kommen, beispielsweise mit einer einstufigen Abkühlung, die der Abtrennung von Methanol und Wasser nachgeschaltet ist. Immer wird jedoch ein gasförmig verbleibender Anteil des Stoffstroms s in einer Absorptionskolonne 16 mit einem überwiegend Dimethylether enthaltenden Rücklauf gewaschen, wie nachfolgend erläutert. Der überwiegend Dimethylether enthaltende Rücklauf wird dabei aus einem bei der Abkühlung abgeschiedenen flüssigen Anteil des Gasgemischs gebildet.

Der Strom s enthält im dargestellten Beispiel noch Dimethylether, Kohlendioxid, Kohlenmonoxid und Wasserstoff, also neben Dimethylether und Kohlendioxid hier zwei leichter als Kohlendioxid siedende Komponenten. Unter Verwendung eines flüssigen Rücklaufs v, der reich an Dimethylether ist und aus einem Teil eines Kondensats z gebildet wird, das aus einem Sumpfstrom aus einer Sumpfflüssigkeit der Dimethylether-Kohlendioxid-Destillationssäule 9 erhalten wird, wird im Sumpf der Absorptionskolonne 16 ein Gemisch aus Dimethylether und Kohlendioxid abgeschieden und in Form des Sumpfprodukts w abgezogen. Das Sumpfprodukt w kann ebenfalls in die Dimethylether-Kohlendioxid-Destillationssäule 9 eingespeist werden. Am Kopf der Absorptionskolonne 16 wird hingegen ein Kopfprodukt x abgezogen, das im Wesentlichen aus Kohlenmonoxid und Wasserstoff besteht und arm oder vorzugsweise frei an Kohlendioxid ist. Dieses wird, gegebenenfalls nach entsprechender Verdichtung in einem Verdichter 17, und abzüglich eines Purgestroms i, zumindest teilweise als Recyclingstrom j, dem Strom b beigemischt.

Der Strom x kann insbesondere dem nicht umgesetzten Synthesegas 916 gemäß Figur 1 entsprechen. Seine Anteile i und j können insbesondere dem Recycle- und Purgestrom 908 bzw. 914 gemäß Figur 1 entsprechen. Eine Verfeuerung in einer Heizeinheit 160 ist in Figur 2 nicht dargestellt.

Der Purgestrom i kann einer zusätzlichen Wäsche mit flüssigem Kohlendioxid unterzogen werden, um möglichst große Anteile des darin enthaltenen Dimethylethers in die Dimethylether-Kohlendioxid-Destillationssäule 9 zurückzuführen. Für diese Wäsche kann vorteilhafterweise ein Teil des kondensierten Kopfgases t aus der Dimethylether-Kohlendioxid-Destillationssäule 9 verwendet werden. Dies ermöglicht eine Verringerung der Verluste an Dimethylether, die andernfalls über die Ausschleusung des Purgestroms i entstünden. Da jedoch nur der Purgestrom i dieser zusätzlichen Wäsche unterzogen wird, wird hierdurch kein zusätzliches Kohlendioxid in den Recyclingstrom j eingetragen. In dem Recyclingstrom j enthaltener Dimethylether wird somit durch den Reaktor 4 in den Trennzyklus zurückgeführt und bleibt somit ebenfalls weitgehend erhalten.

In einigen Ausgestaltungen kann das Kopfgas der Absorptionskolonne 16 in einem optionalen Kopfkondensator 162 kondensiert und zusammen mit dem Strom v als Rücklauf zur Absorptionskolonne 16 verwendet werden. Dadurch wird der Anteil an Dimethylether im Kopfprodukt x reduziert, was zu einem verbesserten Reaktionsgleichgewicht und reduzierten Verlust über den Purgestrom i führt.

In die Dimethylether-Kohlendioxid-Destillationssäule 9 werden, wie erwähnt, der nicht in die Absorptionskolonne 6 rückgeführte Anteil des Stroms I sowie die Ströme q und r und das Sumpfprodukt w eingespeist. Da diese Gemische unterschiedliche Gehalte an Dimethylether und Kohlendioxid aufweisen (Spuren von Kohlenmonoxid und Wasserstoff sind in gelöster Form ebenfalls enthalten), werden diese in unterschiedlicher Höhe in die Dimethylether-Kohlendioxid-Destillationssäule 9 eingespeist, wozu geeignete Ventile (ohne Bezeichnung) vorgesehen sind.

Auch die Dimethylether-Kohlendioxid-Destillationssäule 9 wird mit einem Sumpfverdampfer 91 und einem Kopfkondensator 92 betrieben. Ein aus einem Kopfgas der Dimethylether-Kohlendioxid-Destillationssäule 9 gebildeter Kopfstrom t wird in dem Kopfkondensator 92 unter Verwendung eines mit einem geeigneten Kältemittel betriebenen Wärmetauschers zumindest teilweise verflüssigt und als flüssiger Rücklauf auf die Dimethylether-Kohlendioxid-Destillationssäule 9 aufgegeben. Ein weiterer Anteil wird zur Bildung eines weiteren Stroms y verwendet, der wie Strom 913 gemäß Figur 1 anderweitig verwendet werden kann.

Aus dem Sumpf der Dimethylether-Kohlendioxid-Destillationssäule 9 wird ein flüssiger Strom z entnommen, der hier im Wesentlichen aus Dimethylether besteht, jedoch insbesondere frei oder arm an Kohlendioxid ist. Ein Teil dieses Sumpfstroms z wird zur Bildung des Rücklaufs v für die Absorptionskolonne 16 verwendet. Außerdem wird aus dem Sumpfstrom z das Dimethyletherprodukt gebildet, entsprechend dem Dimethyletherprodukt 911 gemäß Figur 1.

Als der Rücklauf v kommt auch der kondensierte Kopfstrom o der Destillationssäule 5 in Frage, da er im Wesentlichen frei von Kohlendioxid und reich an Dimethylether und ggf. Methanol ist.

Wird Wasser, wie oben in Bezug auf eine Ausführungsform beschrieben, erst stromab der Dimethylether-Kohlendioxid-Destillationssäule 9 abgetrennt, ist es zweckmäßig, einen im Wesentlichen wasserfreien Seitenstrom der Dimethylether-Kohlendioxid-Destillationssäule 9 anstelle deren Sumpfprodukts z als den Rücklauf v für die Absorptionskolonne 16 zu verwenden. Dabei wird der Seitenstrom so gewählt, dass er weniger als 10 Massenprozent, insbesondere weniger als 5 Massenprozent Kohlendioxid enthält.

Der Rücklauf v wird vor der Aufgabe auf die Absorptionskolonne 16 gekühlt, typischerweise auf ein Temperaturniveau, das bei den gewählten Bedingungen etwas über dem Gefrierpunkt von Kohlendioxid liegt, beispielsweise auf ein Temperaturniveau im Bereich von -30 °C bis -49 °C. Zu diesem Zweck kann beispielsweise ein Wärmetauscher 164 vorgesehen sein.

In Figur 3 ist ein Verfahren zur Erzeugung von Dimethylether gemäß einer Ausgestaltung der Erfindung in Form eines vereinfachten Prozessflussdiagramms schematisch dargestellt und insgesamt mit 150 bezeichnet. Zu den jeweils veranschaulichten Komponenten wird auf die Erläuterungen zu Figur 2 verwiesen. Diese können in gleicher oder äquivalenter Weise verwirklicht werden.

Wie in Figur 3 veranschaulicht, wird in dem Verfahren 100 der im Wesentlichen Methanol und Wasser enthaltende Stoffstrom p in die Syntheseeinheit 4 zurückgeführt, insbesondere nach einer Vorbehandlung 30, die eine Verdampfung und Verdichtung auf einen geeigneten Druck umfassen kann. Der Stoffstrom p ist unterbrochen veranschaulicht, wird in einem entsprechenden Verfahren 100 aber insbesondere als kontinuierlicher Stoffstrom geführt. Eine Prozesseinheit zur Einstellung des Verhältnisses von Wasserstoff zu Kohlenmonoxid kann vorhanden sein. In diese kann in Ausgestaltungen der Erfindung ferner nicht gesondert veranschaulichtes Frischwasser eingespeist werden.

## Patentansprüche

1. Verfahren (100) zur Herstellung von Dimethylether, bei dem ein die Reaktanden Kohlenmonoxid und/oder Kohlendioxid und Wasserstoff enthaltendes Synthesegas unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch eine Syntheseeinheit (14, 4) geführt wird, in dem der Methanolkatalysator und der Dimethyletherkatalysator in einer gemeinsamen Katalysezone bereitgestellt sind, wobei der Syntheseeinheit (14, 4) ein Methanol, Dimethylether, Wasser und einen Teil der Reaktanden enthaltendes Rohprodukt entnommen wird, und wobei unter Verwendung zumindest eines Teils des Rohprodukts in einer Trenneinheit (150) eine überwiegend oder ausschließlich Wasser und Methanol enthaltende Fraktion bereitgestellt wird, **dadurch gekennzeichnet, dass** zumindest ein Teil der überwiegend oder ausschließlich Wasser und Methanol enthaltenden Fraktion in einem Wasser und Methanol enthaltenden Rückspeisestrom in die Syntheseeinheit (14, 4) zurückgeführt wird.

2. Verfahren (100) nach Anspruch 1, bei dem der Syntheseeinheit (14, 4) neben dem Synthesegas und dem Rückspeisestrom Frischwasser zugeführt wird.

3. Verfahren (100) nach Anspruch 1 oder 2, bei dem unter Verwendung zumindest eines Teils des Rohproduktstroms in der Trenneinheit (150) eine überwiegend oder ausschließlich die Edukte enthaltende Fraktion bereitgestellt wird, die ebenfalls zumindest zum Teil in die Syntheseeinheit (14, 4) zurückgeführt wird.

4. Verfahren (100) nach einem der vorstehenden Ansprüche, bei dem die Syntheseeinheit (14, 4) einen ersten Reaktor oder Teilreaktor und einen zweiten Reaktor oder Teilreaktor umfasst, wobei zwischen dem ersten Reaktor oder Teilreaktor und dem zweiten Reaktor eine Abscheideeinheit verwendet wird, unter Verwendung derer ein Partialdruck von Wasser und Kohlendioxid reduziert wird.

5. Verfahren (100) nach einem der vorstehenden Ansprüche, bei dem das Synthesegas zu einem ersten Anteil unter Verwendung einer Vergasungseinheit (20) bereitgestellt wird, und bei dem das Synthesegas ferner separat von der Vergasungseinheit (20) bereitgestellten und/oder unter Verwendung einer Wassergasshiftreaktion bereitgestellten Wasserstoff umfasst.

6. Verfahren (100) nach Anspruch 5, bei dem der separat von der Kohlenwasserstoffvergasungseinheit (20) bereitgestellte Wasserstoff zumindest zu einem Teil mittels Elektrolyse bereitgestellt wird.

7. Verfahren (100) nach einem der vorstehenden Ansprüche, bei dem die überwiegend oder ausschließlich Wasser und Methanol enthaltende Fraktion einen Summengehalt von Wasser und Methanol von mehr als 90% aufweist.

8. Verfahren (100) nach einem der vorstehenden Ansprüche, bei dem die überwiegend oder ausschließlich Wasser und Methanol enthaltende Fraktion unter Verwendung einer Absorptionskolonne (6) und einer Destillationssäule (5) bereitgestellt wird, wobei der Absorptionskolonne (6) zumindest ein Teil des Rohprodukts zugeführt wird, ein überwiegend leichter als Methanol und Wasser siedende Komponenten enthaltender Kopfstrom der Absorptionskolonne (6) zu einem Teil kondensiert und als Rücklauf auf die Absorptionskolonne (6) zurückgeführt wird, ein überwiegend Methanol und Wasser sowie Kohlendioxid enthaltender Sumpfstrom der Absorptionskolonne (6) in die Destillationssäule (5) eingespeist wird, und die überwiegend oder ausschließlich Wasser und Methanol enthaltende Fraktion unter Verwendung eines Sumpfstroms aus der Destillationssäule (5) bereitgestellt wird.

9. Verfahren (100) nach einem der vorstehenden Ansprüche, bei dem der Rückspeisestrom zumindest ein Teil der überwiegend oder ausschließlich Wasser und Methanol enthaltenden Fraktion enthält, der einer Verdampfung und Druckbeaufschlagung unterworfen wird.

10. Verfahren (100) nach Anspruch 9, bei dem die Druckbeaufschlagung vor der Verdampfung mittels einer Pumpe durchgeführt wird.

11. Verfahren (100) nach Anspruch 9 oder 10, bei dem die Verdampfung zumindest zum Teil ein Durchleiten eines heißen Gasstroms durch die überwiegend oder ausschließlich Wasser und Methanol enthaltende Fraktion umfasst.

12. Verfahren (100) nach Anspruch 9, bei dem die Druckbeaufschlagung nach der Verdampfung mittels eines Verdichters durchgeführt wird.

13. Verfahren (100) nach einem der vorstehenden Ansprüche, bei dem das Synthesegas mit einem Verhältnis von Wasserstoff zu Kohlenmonoxid von weniger als 2 bereitgestellt wird.

14. Anlage zur Herstellung von Dimethylether, mit einer Syntheseeinheit (14, 4) und Mitteln, die dafür eingerichtet sind, ein die Reaktanden Kohlenmonoxid und/oder Kohlendioxid und Wasserstoff enthaltendes Synthesegas unter teilweiser Umsetzung der Reaktanden und Bildung von Methanol an einem oder mehreren Methanolkatalysatoren und Umsetzung zumindest eines Teils des Methanols an einem oder mehreren Dimethyletherkatalysatoren durch die Syntheseeinheit (14, 4) zu führen, wobei in der Syntheseeinheit (14, 4) der Methanolkatalysator und der Dimethyletherkatalysator in einer gemeinsamen Katalysezone bereitgestellt sind, und wobei die Anlage Mittel aufweist, die dafür eingerichtet sind, aus der Syntheseeinheit (14, 4) ein Methanol, Dimethylether, Wasser und einen Teil der Reaktanden enthaltendes Rohprodukt zu entnehmen und unter Verwendung zumindest eines Teils des Rohprodukts in einer Trenneinheit (150) eine überwiegend oder ausschließlich Wasser und Methanol enthaltende Fraktion bereitzustellen, **dadurch gekennzeichnet, dass** die Anlage dafür eingerichtet ist, zumindest einen Teil der überwiegend oder ausschließlich Wasser und Methanol enthaltenden Fraktion in einem Wasser und Methanol enthaltenden Rückspeisestrom in die Syntheseeinheit (14, 4) zurückzuführen.
